# EUROPEAN PATENT APPLICATION

(11) **EP 4 205 540 A1**
(43) Date of publication of application: **05.07.2023**
(21) Application number: 21218485.7
(22) Date of filing: 31.12.2021
(51) Int. Cl.: A01K 67/033

(54) **METHOD AND ARRANGEMENT FOR REARING INSECT LARVAE**

(71) Applicant: Manna Insect Oy, 90650 Oulu (FI)
(72) Inventor: Marjanen, Ykä, 90650 Oulu (FI); Pyörälä, Jari-Pekka, 90650 Oulu (FI); Haukkala, Eerik, 90650 Oulu (FI)
(74) Representative: Kolster Oy Ab

(57) **Abstract**

A system for rearing black soldier fly larvae comprises a container (150); multiple trays (110) for rearing the larvae; at least one sensor (132) for one of the trays used as a reference tray, configured to measure at least moisture of a biomass in said reference tray; at least one temperature sensor (130, 126) to measure temperature in the reference tray and/or air temperature in the container; means (140) for controlling, dynamically in response to the measured moisture and temperature of the biomass, temperature and humidity inside the container to maintain the temperature in the biomass within a determined range and to maintain the measured moisture in the reference tray below a level where liquid separate from the biomass, and to control, upon completing the rearing, at least one of the temperature and humidity in the container to dry a remainder of the biomass, frass, and the larvae.

## Description

### TECHNICAL FIELD

The present invention relates to a field of industrial rearing and breeding of insect larvae, in particular black soldier flies.

### TECHNICAL BACKGROUND

Black soldier fly larvae are used to compost bio waste and convert the bio waste into animal feed. The larvae are among the most efficient animals at converting biomass into feed. The black soldier fly larvae can be used as an alternative source of protein for aquaculture, animal feed, pet food and human nutrition, especially replacing soy and fish meal. Such alternative sources of protein and their industrial-scale production is one of key factors in combatting climate change and allowing animal growers a more local method to produce animal feed.

### BRIEF DESCRIPTION

The invention is defined by the independent claims. Various embodiments are provided in the dependent claims.

### BRIEF DESCRIPTION OF THE DRAWINGS

In the following the invention will be described in greater detail by means of preferred embodiments with reference to the accompanying drawings, in which
Figure 1 illustrates a larvae rearing system according to an embodiment;
Figure 2 illustrates a process for controlling environmental conditions in a larvae rearing container according to an embodiment;
Figure 3 illustrates another process for controlling environmental conditions in a larvae rearing container according to an embodiment;
Figure 4 illustrates an arrangement for collecting escaping larvae;
Figure 5 illustrates control profiles for controlling the environmental conditions during the rearing.

### DESCRIPTION OF EMBODIMENTS

The following embodiments are exemplifying. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments.

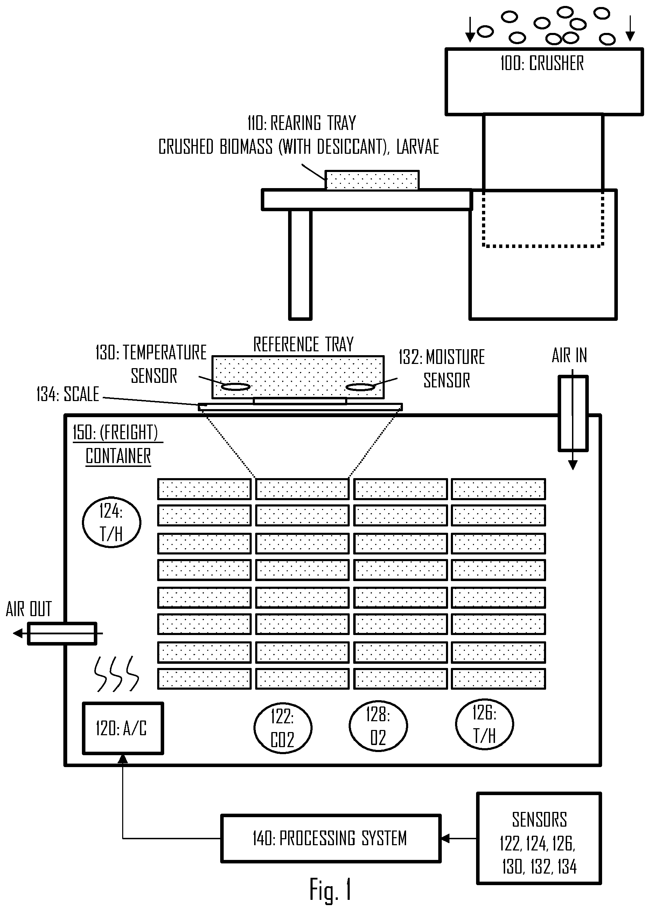

Figure 1 illustrates an embodiment of a larvae rearing system to which industrial processes for larvae rearing according to the embodiments described below can be implemented. The larvae rearing may be carried out in a freight container 150 that may be a standard-sized (ISO, International Standardization Organization) container. An advantage of using such a freight container is easy transportation to various locations on the Earth. The larvae rearing system comprising any or all the structures and components described herein may be inserted into the container for the transportation and transported to the site where the larvae rearing is to be performed.

The larvae rearing system may comprise, in the container, one or more sensors 122, 124, 126 to measure various environmental conditions in the container, such as air temperature, air humidity, and one or more gas sensors for measuring an amount of oxygen and/or carbon dioxide in the air inside the container. The container 150 may further comprise a ventilation system to allow inlet of fresh air into the container from outside and outlet of air from the container to maintain, for example, a certain oxygen level in the container for the larvae or to increase the oxygen level. The container may further comprise an air conditioning system 120 configured to control the temperature and humidity in the container under the control of a processing system 140. The processing system 140 may comprise at least one processor and at least one memory storing computer program instructions of a computer program product that, when read and executed by the at least one processor, cause the processor to carry out a computer process comprising control actions described in the embodiments below. The processing system may be a local computer in the container 150 or a remote computer external to the container but in communication with sensors and climate control devices (the air conditioning system 120, the ventilation system, etc.) in the container. The communication may be realized via wired and/or wireless communication protocol(s). In an embodiment, the processing system comprises a server computer that employs cloud computing. The processing system 140 may acquire measurement data from the sensors 122 to 126 and also from further sensors described below and control the air conditioning system and/or ventilation on the basis of the measurement data and a control profile designed for the larvae rearing.

The container may comprise shelves or other support structures for a plurality of trays for rearing the larvae. The trays comprise substantially the same composition of preprocessed (e.g. crushed) and a homogenous biomass of larvae food, black soldier fly (*hermetia illucens*) larvae, and possibly a desiccant to absorb moisture extracting from the biomass during the rearing, if required. The principles described below are applicable to other insect larvae as well. The desiccant may be readily in the biomass during the crushing in the crusher 100, or it may be added to the biomass after the crushing. The desiccant may be biomass as well, such as grain or fodder. With some forms of the crushed biomass, no desiccant is needed but with potatoes, for example, the desiccant helps the biomass to stay sufficiently moist by absorbing liquid extracting from the crushed potatoes, and combining it with a ventilation system and a dryer will allow to control air and bio waste humidity.

The biomass may be biowaste. By using the freight container, the larvae rearing system can be brought next to the source of the biowaste instead of transporting the biowaste.

With respect to the 'recipe' of each tray for the larvae, designed for the biomass being crushed potatoes, a mass of the black soldier fly larvae (at an age of five to seven days or six to seven days) is about 0.00003 to 0.00004 weight percent of the biomass. For example, if the tray contains seven kilograms crushed potatoes, the amount of desiccant may be about 300 to -400 grams of desiccant fodder, and 0.25 grams of black soldier fly larvae egg weight, that is pre-grown to 5-7 days old larvae. The size of each tray may be, for example, 600x400x145 millimetres (length, width, height). This is an example of a recipe found suitable for rearing large larvae and having a short rearing duration.

The number of such identical trays in the container may be in the order of dozens or even hundreds, depending on the size of the trays and the size of the container. Each tray may be prepared by crushing (or severing) the biomass into such a biomass that is suitable for consumption by the larvae, in particular the black soldier fly larvae. The larvae do not have teeth, so the biomass is advantageously crushed so that it is better edible to the larvae. This speeds up their growth and reduces the duration of the rearing. In an embodiment, the homogenous biomass is mostly made of root vegetables and includes potatoes. In some embodiments, the homogenous biomass is mostly or even solely potatoes. The constitution of the biomass may be substantially equal in all trays, and the trays may have the same size and shape. Potato is an excellent source for the biomass because of high nutritional value for the larvae. A problem with root vegetables (or any other type of fruits or vegetables) and particularly potatoes is that, when crushed in a crusher 100 and put into the trays together with the larvae, they start extracting liquid (water). The extraction of the liquid continues while the larvae consume the biomass. The water is necessary for the purpose of moistening the biomass so that it is edible to the teethless larvae. The biomass needs to be very moist for that purpose. However, the liquid should not separate from the biomass so that the tray would not flood. In case of flooding, the larvae will start to escape from the tray and will affect the eating of the larvae and, thus, the growth of the larvae. The conditions inside the container naturally affect the amount of flooding in the tray, particularly temperature and humidity.

An embodiment of the invention uses one of the rearing trays as a reference tray 110 provided with a sensor system to measure at least moisture (moisture sensor 132) of the biomass regularly during the rearing. Additionally, temperature in the reference tray measured in an embodiment (temperature sensor 130). As an alternative to the temperature sensor in the reference tray, a temperature sensor 126 measuring air temperature in the container may be used. Measuring the temperature of the biomass in the reference tray may provide a more accurate estimate of the true rearing temperature experienced by the larvae, because the larvae will also generate heat. The sensors 126, 130, 132 are also connected to the processing system 140. Thanks to the rearing trays being identical in their dimensions (size, shape) and their contents, measuring one of the trays provides a substantially accurate estimate of the conditions in every rearing tray inside the container. There may be multiple trays for rearing the larvae, in addition to the reference tray(s), wherein the multiple trays may either have dimensions and contents that are identical to dimensions and contents of the reference tray(s) or have dimensions and contents proportional to the dimensions and contents of the reference tray(s) such that they provide the same response to the temperature and humidity control as the reference tray. For example, the depth of the trays and the depth of the biomass in the trays, and the recipe of the biomass and the eggs or larvae may be identical but a surface area of the trays may vary. Typically, it may be more efficient to provide only identical trays in the container. The support structures or shelves for the trays may be arranged to allow air circulation between the trays and between the interiors and exteriors of the trays so that the air conditioning can affect both the air inside the container and the temperature and moisture of the biomass where the larvae is disposed. Depending on the biomass, there might be more than one reference trays to improve accuracy of the measurements and analysis. One or more sensors inside the container will measure the air flow and the temperature and humidity distribution inside the container, which can be further used to extrapolate conditions in all trays based on measuring one or multiple reference trays.

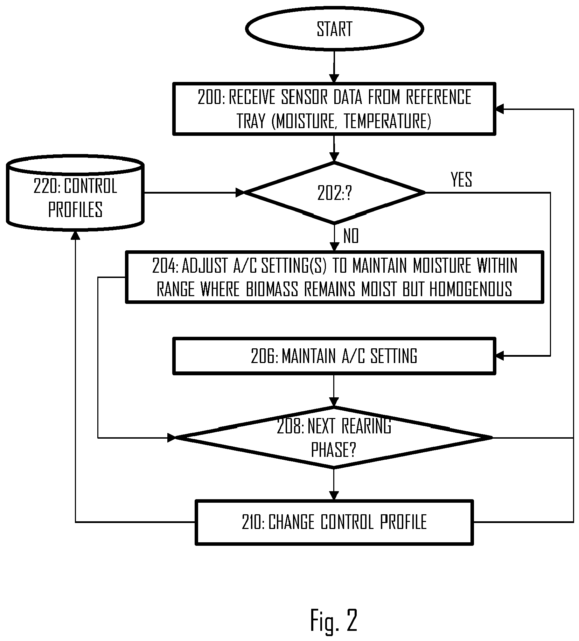

The rearing method and the respective system or arrangement comprises means for controlling the moisture of the biomass and temperature of the air and/or biomass within optimal range. One embodiment of such means is the desiccant that may be added to the biomass to dry the biomass and to absorb the liquid extracting from the biomass such as the crushed potatoes. In another embodiment, the conditions inside the container and in the trays are controlled on the basis of the measurement data received from the sensor(s) in the reference tray and, optionally, sensors in other parts of the container, e.g. the temperature sensor measuring air temperature of the container. Figure 2 illustrates an embodiment of such a procedure for controlling the conditions in the container. The process may be carried out as a computer-implemented process by the processing system 140. Referring to Figure 2, the process comprises receiving in block 200 the measurement data from the sensors 122, 124, 126, 130, 132, 134 or a subset thereof, e.g. from the sensors of the reference tray. The communication between the sensors and the processing system may be carried out according to wired or wireless communication protocols and networking solutions known in the art. There exist several wireless communication protocols suitable for the purpose, e.g. Bluetooth, Wifi (IEEE 802.11) or Zigbee, and detailed description of them is omitted here.

In block 202, the measurement data (temperature measurement data, measured moisture of the biomass) is compared with one or more thresholds that are defined by a currently applicable control profile retrieved from a memory 220. If the comparison in block indicates that the threshold is exceeded, indicating that adjustment to the conditions is needed, the temperature, humidity etc. is adjusted in block 204 dynamically in response to the measured moisture of the biomass and temperature of the biomass and/or air. The temperature and humidity inside the container are controlled to maintain the moisture and temperature in the biomass within a determined range, as defined by the threshold(s) of the control profile 220, and to maintain the measured moisture in the reference tray below a level where liquid (water) separates from the biomass. In an embodiment, the measured moisture is further maintained above a determined threshold defining a lower boundary for optimal rearing, e.g. 60% relative humidity (RH), to keep the moisture high enough so that the larvae is able to consume the biomass. The level where the liquid separates from the biomass may be measured by using the moisture sensor. With commercially available sensors arranged to measure soil, for example, a certain reading representing the moisture is acquired as the measurement data, and the reading may be cross-referenced to ranges mapped to certain conditions of the biomass. One range may be for indicating saturation of the moisture such that the biomass is heterogenous and contains the liquid extracted from the biomass. This range may be different for different compositions of the biomass and may depend on, for example, a degree of porosity of the biomass, just as with the soil. One range may be for indicating that the moisture is appropriate for the rearing, e.g. between a reading of a saturation point and a reading at a threshold for the biomass being too dry, and one range for indicating that the biomass is too dry. Different ranges may be provided for different recipes, e.g. for different contents of the biomass. The skilled person is able to find the suitable ranges for the different compositions of biomass by experimentation. If it is determined in block 202 that the measurement data indicates conditions being within a suitable range, the process proceeds to block 206 where the current settings for the conditions are maintained. In this manner, the conditions are dynamically controlled during the rearing. In block 208, it is determined whether or not a new phase in the rearing is reached. Embodiments of the phases are described below. If the new phase is determined to have started, e.g. as determined on the basis of the measurement data or on the basis of a time elapsed for the current phase, the process proceeds to block 210 where a new control profile is selected from the memory 220, comprising at least one different setting for the conditions with respect to the phase that has ended. Thereafter, block 202 employs at least partially different threshold for controlling the conditions.

While eating, the larvae is also responsible of evaporating the moisture from the food. It is important to measure and know the larvae growth stage and to inhibit it to grow too fast at the critical time, or to predict and prepare for it with ventilation and drying. Otherwise, water will be evaporated too fast and thus becomes liquid in the box. At a critical time, the larvae are big enough to cause significant evaporation while also the biomass is still too wet. It is also important to monitor larvae growth for alarming the operator if larvae do not grow as predicted. This may also cause changes to the control profile, and these changes may be made manually by the operator.

In an embodiment the temperature is maintained between 28 and 33 degrees centigrade during the rearing phase. The relative humidity of the air may be maintained between 55 and 80 per cent RH. At these ranges, optionally together with other parameters during the rearing enable suitable conditions for the larvae to feed and grow fast, and to remain in the trays. Optimal growth and rearing results in greater size of the larvae, lower energy consumed, less escaping, and less larvae morphing into pupas during the rearing. Additionally, the carbon dioxide (CO2) level may be controlled such that the control settings change based on the larvae growth. An optimal value may depend on the larvae growth stage and be between 400 parts per million (ppm) to 8000 ppm.

As described above, the oxygen and/or carbon dioxide levels may also be monitored during the rearing in order to maintain the air quality suitable for rearing. As the larvae grows, they consume more oxygen. On the other hand, increasing carbon dioxide levels may cause the larvae to stop feeding or even try to escape the trays. Therefore, maintaining the oxygen and carbon dioxide levels within predefined ranges during the rearing further improves the rearing while minimizing unnecessary energy consumption. The moisture of the biomass and oxygen and carbon dioxide levels may be controlled by the processing system by controlling the ventilation on the basis of the measurement data received from the sensors 122, 128. Filter(s) such as active carbon filter(s) may be provided at least in the air outlet to filter the air exiting the container, thus reducing the smells around the container. The ventilation control may employ weather forecasts. For example, the ventilation may be carried out on a greater level during daytime or during warm days and less during nighttime or during colder days. If it is estimated that the external temperature increases, increasing the ventilation may be postponed until the higher temperatures realize. Similarly, if the temperature is expected to decrease and the measurement data from the sensors 122,128 indicate a need for ventilation, full ventilation may be conducted before shutting down or at least decreasing the ventilation before the lower temperatures realize. The inlet air may be pre-heated before directed to the interior of the container.

In summary, the moisture of the biomass may be controlled by at least the following means: the ventilation via the ventilation system, humidity control via the air conditioning system 120, and by using the desiccant. Any one or more of these means may be used during the rearing. With a certain recipe of the biomass, the desiccant may be even omitted. The ventilation may be preferred over the use of the air conditioning system because of lower power consumption of the ventilation system. As a consequence, the control profile may be designed such that, upon detecting that the measured moisture of the biomass exceeds a first threshold, the processing system 140 may start the ventilation system or increase its ventilation power. If the measured moisture does not drop below the first threshold within a determined time interval from the detection and following ventilation system control action, or if the measured moisture exceeds a second threshold greater than the first threshold, the processing system 140 may control the air conditioning system 120 to start drying the air inside the container, thus also reducing the moisture of the biomass.

One solution for reducing or preventing the larvae to escape is a mechanical collar or a similar solution for each tray. The collar may be designed such that it directs the larvae climbing on the walls of the tray to fall back to the biomass in the tray.

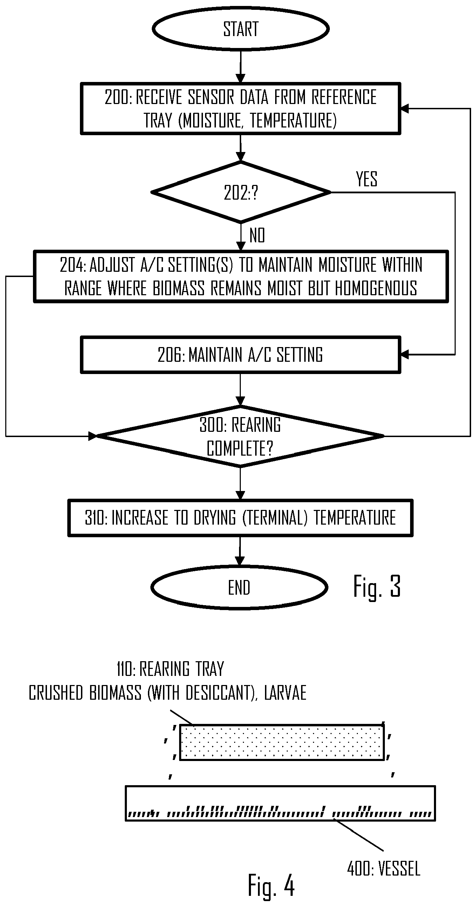

An embodiment of the new phase and the new control profile is that, upon completing the rearing, the temperature in the container is increased to dry the trays for harvesting the larvae. At this stage, the trays contain the remainder of the biomass not consumed by the larvae, frass extracted by the larvae, and the larvae itself. This is illustrated in Figure 3 that is a simplified version of the procedure of Figure 2. With the embodiment of Figure 2, an arbitrary number of phases with respective control profiles may be implemented, e.g. according to the embodiments described below. In the embodiment of Figure 3, one control profile may be used until the rearing is determined to be complete in block 300. If the rearing is not completed, steps 200 to 206 may be iterated in the above-described manner. If the rearing is determined to be completed, the process proceeds to block 310 where the temperature in the container is increased to a level that is greater than an allowed maximum temperature in block 202. As a consequence, a temperature control threshold may be raised to a higher level to implement the drying. Furthermore, a humidity threshold may be lowered. In an embodiment, the temperature is raised to 60 degrees centigrade or above, e.g. 70 degrees centigrade, for at least one hour, thus providing the following effects on the trays: the biomass is dried, the larvae are terminated, and the contents of the trays are sanitized for intermediate storage, for example. The drying results in that it is much easier to separate the larvae from the remaining biomass, because the larvae naturally separate from the biomass. As a consequence, cleaning the trays is simplified or can even be omitted. In another embodiment, the temperature is raised but to a lower level, e.g. to a level that is below 40 degrees centigrade to realize the drying of the biomass and the frass without terminating the larvae. Another aspect in the drying phase is to increase the ventilation upon completing the rearing, and the ventilation may be used instead of the temperature increase or in addition to it to improve the drying effect.

As described above, block 300 may be a step within block 208, e.g. for determining the final phase of the rearing. In an embodiment, the decision in block 300 is based on the received measurement data. One of the sensors in the system of Figure 1 is a scale configured to measure a mass of the reference tray. As the larvae grows, the biomass is eaten and water evaporates, the total mass decreases, while mass temperature rises close to air temperature and mass moisture decreases dramatically. During the rearing, the growing larvae will generate heat, thus reducing the need for generating heat to maintain the desired temperature in the container. The temperature threshold may be even lowered towards the end of the rearing while the rearing is still going on. The reason is that growing larvae can sustain lower temperatures, thanks to their greater size. Since the recipe is known or can be measured with sensors, a threshold for the mass can be defined for use in block 300 for determining whether or not the rearing is complete. When the measured mass received by the processing system from the scale directly or via manual input by an operator is determined to be above the threshold, the process may proceed to block 310 and the final drying/termination phase may be triggered.

During the drying phase and, particularly, in the terminating phase, it is possible that some or even most of the larvae will escape the trays. For that purpose, a vessel may be provided below each of the trays to collect the larvae that escape from the trays. Accordingly, the larvae may be harvested from the vessels and from the trays after said termination, thus separating the larvae from the biomass. Figure 4 illustrates a rearing tray 110 with a vessel 400 below the tray. The dimensions of the vessel may be designed so that the larvae escaping over the edges of the tray will fall into the vessel. The vessel may be large enough with respect to the tray so that it collects the larvae that escapes over all edges of the tray. Such a vessel may be provided under each tray. In case the trays are piled on top of one another in the support structure or shelves, the support structure may be designed so that the larvae will fall either into a tray below or to the vessel under the pile of trays on the floor level, for example.

After the terminating phase, the processing system may output a notification to indicate to an operator that the rearing is complete and that the larvae may be harvested from the vessels and/or from the trays. Thanks to the drying, the larvae can be easily separated from the dried biomass, for example, by using a sieve. Thereafter, the harvested larvae may be subjected to post-processing such as chopping outside the container. The larvae may be stored directly after drying as the temperature at the end exceeds sanitation requirements of 70 degrees Celsius for one hour.

The procedures of Figures 2 and 3 are fully automated, and the rearing can be performed even without opening the freight container doors during the entire rearing season that may span over two weeks, for example. An advantage in this is obviously low need for manual inspection and control but, additionally, improved rearing because no light may be needed inside the container. The larvae react to the light and may frighten, thus reducing their appetite and degrading the rearing.

In the same embodiment, operator can give an instruction for a specific date or duration of the rearing period. The system can optimize temperature, humidity and other parameters so that the larvae are optimally ready for harvesting and separating at that exact time. Also, as the larvae in some cases are used for breeding, it is beneficial to optimize the rearing process to produce pupae as quickly as possible. Again, the system can optimize conditions to allow this to happen automatically.

In an embodiment, the sensor system further comprises an imaging sensor is directed to monitor a surface of the biomass in the reference container. The image sensors may be an infrared imaging sensor, for example, and the image sensor may also be coupled with the processing system. By using the measurement data (images) from the image sensor, motion of the larvae is monitored by analysing the images captured by the imaging sensor. In an embodiment of block 202, the process comprises triggering a control action to reduce the moisture upon detecting in the image analysis that the motion of the larvae increases a threshold. Increased number of larvae in the surface or sides of the tray is an indicator that the larvae is not consuming the biomass but, instead, trying to escape from the tray, which is a direct indicator of too high moisture of the biomass, or an issue with other condition parameters such as too hot temperature or too low oxygen levels. The image analysis may be combined with the readings from the moisture, temperature and CO2 sensor to determine when the biomass is too wet for efficient rearing. Accordingly, the processing system may use two inputs for increasing the drying in the container: 1) the drying may be triggered or increased (by controlling the air conditioning system) upon detecting on the basis of the moisture sensor readings that the biomass is too wet; 2) the drying may be further increased upon detecting in the image analysis increased motion (above a threshold) in the larvae.

The processing system may employ the image analysis for other purposes as well. Imaging the reference tray and the activity of the larvae in the reference tray provides various information, in addition or as an alternative to evaluating the moisture of the biomass. The image analysis may be used to estimate when the biomass has been consumed by the larvae. The biomass can be separated from the larvae in the image analysis by conventional pattern recognition and classification methods. The image analysis may be used to measure the growth of the larvae, by using conventional pattern recognition and size estimation from the received image data. Certain reference values may be defined for target growth, and the size of the larvae determined from the images may be compared with the reference values. The processing system may output a notification or an alarm in one or more of the following cases: the size of the larvae is smaller than the size represented by the reference values; there is no movement or movement below a certain threshold level is detected in the image analysis (an indicator of that the larvae has either escaped or died). In another embodiment, the processing system may use the image analysis to estimate the size of the larvae.

Using image analysis with a combination of tray weight and other optionally sensors, the conversion rate of the biomass to larvae, larvae weight and readiness to be harvested can be determined.

In an embodiment, the measured weight is used to monitor the escaping rate of the larvae. For example, if the weight drops below a determined threshold, the processing system may output a notification or an alarm to indicate that the escaping rate may be too high. In response to the notification or alarm, the operator may carry out inspection of the trays, larvae, and rearing.

It is also possible that the size of the larvae is above the reference values. It means that the rearing has exceeded its targets in terms of the growth of the larvae. In such a case, the processing system may store a record of the control actions and the measurement data acquired during the rearing, and link a notification of successful rearing to the record so that the same profiles may be used in future rearing seasons. In this manner, the processing system may employ machine learning to find optimal conditions for the rearing. The processing system may employ other machine learning characteristics during the rearing. The processing system may, for example, experiment outside the thresholds of the currently applicable control profiles and measure the 'reward' of the experiments by using the sensors of the reference tray. For example, the processing system may increase the temperature over the upper threshold during the rearing, e.g. for a duration of one day, and measure the effect of that increase in the activity of the larvae and/or in the size of the larvae after the experimentation or after the rearing.

In general, all the measurement data and control actions during the rearing season may be stored in a record. After the rearing, a questionnaire may be provided to operator so that the operator may enter the results of the rearing, such as: the size of the larvae, the weight of the larvae, the portion of dead or escaped larvae, etc. The results may then be added to the record. Upon gathering such records over numerous (e.g. at least two) of rearing seasons (in one or in multiple containers in parallel rearing), the processing system may analyse the data and optimize the control profiles. The same optimized control profile may then be used in various containers that may be disposed arbitrarily. For example, if the processing system employs a central server (cloud computing) and local processing systems in each container, the central server may store the control profiles and, upon starting a new rearing season in a container, the local processing system of the container may retrieve the control profiles from the central server over an Internet connection. In this manner, a continuously learning and evolving automated rearing system can be established. The more containers and rearing seasons have been carried out, the more can the processing system learn from past experiences and optimize the control profiles. This results the advantage that the environmental conditions for the rearing can be constantly improved, thus providing shorter rearing times, less escaping of the larvae, and improved yield of the larvae, while consuming less electrical energy. The yield refers to that less larvae or eggs are needed to provide the same amount of larvae at the end of the rearing.

Yet one aspect of the machine learning comprises estimating the characteristics of the container with respect to controlling the conditions in the container. As described above, the purpose is to automatize the rearing process so that the conditions are maintained within the threshold(s) specified for each control profile. The container may, however, be different for different rearing seasons and, therefore, the processing system may test the characteristics in the beginning of the rearing and, in some embodiments, during the rearing. One aspect of testing the characteristics also during the rearing may be to detect faults in the container, such as holes or other problems in the insulation, or malfunctioning of a sensor or a control system such as the air conditioning system or a heater. Such testing can of course be made between the rearing seasons per container but, in optimal utilization, a time interval between consecutive rearing seasons may be in the order of an hour or a few hours, while the rearing season may span for over a week, e.g. two weeks. One aspect is to measure 'inertia' of the container to respond to adjustment of temperature or humidity control. This may be carried out by the processing system by instructing the air conditioning system or the ventilation system to change the temperature or humidity (increase or decrease) and to measure a response time by using the temperature or humidity sensor(s). Predetermined reference response time(s) may be defined and, upon measuring the response time, the processing system may determine whether or not the response time is within an acceptable tolerance. External conditions outside the container may be taken into account, e.g. on the basis of weather forecast information or by using sensors outside the container. For example, when it is cold outside a longer response time may be allowed when the response time relates to increasing the temperature inside the container. Similarly, when it is hot outside the container, a longer response time may be allowed for cooling the container. The effect of the ventilation may be tested by inputting a command to the air conditioning system to change the temperature/humidity and by measuring the temperature/humidity in different parts of the container, by using the available sensors. If the sensors indicate that the temperature/humidity changes in substantially similar manner in the different parts of the container, similarity determined by defining certain tolerances, the air conditioning may be determined to operate suitably. On the other hand, if the sensor data indicates that the temperature/humidity differs or changes differently in the different parts of the container, the processing system may determine to either increase the ventilation or to report a notification of a malfunctioning ventilation system. One input parameter for testing may define whether the container is empty or filled with trays, and different acceptable response times or other reference parameters may be defined.

As described above, the rearing may be divided into multiple phases (e.g. two or more phases) with different control profiles (settings) for controlling the conditions in the container. In an embodiment, the humidity control during the rearing follows the following pattern: during a first time interval from a start of the rearing, the humidity control is drying to prevent the liquid from separating from the biomass; during a second time interval following the first time interval, the humidity control is humidifying to prevent the biomass from drying; during a third time interval following the second time interval and associated with completing the rearing, the humidity control is to dry the biomass and the larvae. This is possible with the presented solution where all the larvae in the container is at the same age at the time of starting the rearing and, as a consequence, grow and experience the environmental conditions in the same manner.

Similarly, the oxygen volume control, temperature control, and carbon dioxide control may be divided into multiple phases, each phase having a unique set of one or more thresholds for controlling the conditions in the container and in the rearing trays. Figure 4 illustrates an embodiment of the different parameters that are controlled during the rearing. The control profiles for each parameter (oxygen O2, humidity, carbon dioxide CO2, temperature) are illustrated in separate graphs, but they can be understood to be along the same timeline, so they are on scale with respect to one another. Next, let us describe the different phases with reference to Figure 4, first separately for each parameter and then how they are aligned together. In each graph, the solid line represents the values of the respective parameters while the dashed lines represent the thresholds (upper and lower) for the control profile of the respective parameters. In other words, the dashed lines represent ranges within which the respective parameters are maintained under the control of the processing system.

Now, referring to the oxygen volume control during the rearing, the oxygen volume control follows the following pattern: the relative volume of oxygen in the container or input of the oxygen into the container is increased towards the end of the rearing and decreased upon completing the rearing. The reason for increasing the oxygen input is that the growing larvae will consume more oxygen and, therefore, its input needs to be increased. This can be carried out in multiple ways: by increasing the oxygen input directly, increasing ventilation, or by decreasing carbon dioxide levels in the container. The reason for decreasing or even stopping the oxygen input upon completing the rearing is that the larvae are stunned or rendered unconscious before terminating them in the final phase. The reason for optimizing ventilation and oxygen control is also to minimize unnecessary energy consumption. With minimal energy consumption it is possible that the container and control system is fully powered by a solar panel system. Also, by controlling oxygen with temperature the growth of larvae can be controlled to optimize the duration of the rearing period based on the need.

Referring to the humidity control, the humidity control follows the above-described pattern illustrated the graph directly below the top-most graph. The humidity illustrated in Figure 4 may represent air humidity in the container. In the first phase (the drying phase), there is more separation of liquid from the potatoes, or other bio waste, because the potatoes have just been crushed and, therefore, the humidity control is drying the air. Thereafter, the setting is changed so that greater humidity is allowed so that the biomass remains moist. However, the drying feature is maintained for the situation that the measurements (moisture, image analysis) indicate flooding in the reference tray. Upon determining that the rearing is complete, e.g. on the basis of the mass of the reference tray reaching a threshold, the humidifying turns to drying again the dry out the biomass and, thereby, separate the larvae from the biomass.

With respect to the carbon dioxide volume control during the rearing, the carbon dioxide volume control follows the following pattern: the relative volume of carbon dioxide is increased upon completing the rearing to stun the larvae before carrying out the increase of the temperature. This is illustrated in the second graph from the bottom of Figure 4. As described above, the CO2 control may relate to the O2 control in the sense that as the relative volume of oxygen is decreased, the volume of the carbon dioxide is increased. This can be realized by stopping the ventilation so that the larvae will consume the oxygen and exhale the carbon dioxide, thus increasing the carbon dioxide levels. Similar pattern can be done using oxygen sensor or e.g. ammonium sensor, which indicates metabolism of the larvae.

The bottom-most graph in Figure 4 represents temperature control during the rearing. The temperature control follows the following pattern: the temperature is maintained within a determined range during the rearing and, upon completing the rearing, the temperature is first decreased below the determined range before said increasing the temperature above the determined range. The reason for decreasing the temperature upon determining that the rearing is complete is that the decrease slows down the larvae, thus reducing the probability of the larvae escaping from the trays when the carbon dioxide levels increase. And as described above, the purpose of increasing the temperature is to both dry the biomass, to terminate the larvae, and to sanitize the trays and their contents. Temperature is also optimize based on the set target duration of the rearing process. If longer period is requested, then temperature is lowered to reduce metabolism (growth) of the larvae. Additionally, temperature is also optimized based on outside weather to prevent using excess energy.

Let us then iterate the control sequence upon determining that the larvae rearing is complete. The first step may be starting the drying phase by reducing the humidity in the container. At the same time or after the drying has been conducted for a certain time, the temperature may be decreased below the determined range before increasing the relative volume of carbon dioxide. After the temperature has reached a certain target temperature, the processing system may start increasing the carbon dioxide levels. Thereafter, the temperature may be increased above the determined range, e.g. to over 70 degrees centigrade, to terminate the larvae. By using such a control profile, together with the vessel(s) below the trays, as high as 90% of the larvae has been successfully separated from the biomass and the trays into the vessels at the end of the rearing. As a consequence, only a minor mechanical separation needs to be conducted on the larvae remaining in the trays. And trays don't need to be washed as dry biomass and larvae separate over 95% and everything is sanitized with temperature.

In some embodiments, the reference tray may be omitted. For example, the reference tray may be used until the suitable control profiles for the rearing have been reached according to the learning principles described above. In such a case, keeping the conditions (size of the trays, the recipe etc.) constant enables that the optimal conditions can be maintained even without measuring the reference tray. For example, the control profiles described may be carried out without the reference tray and respective measurement data.

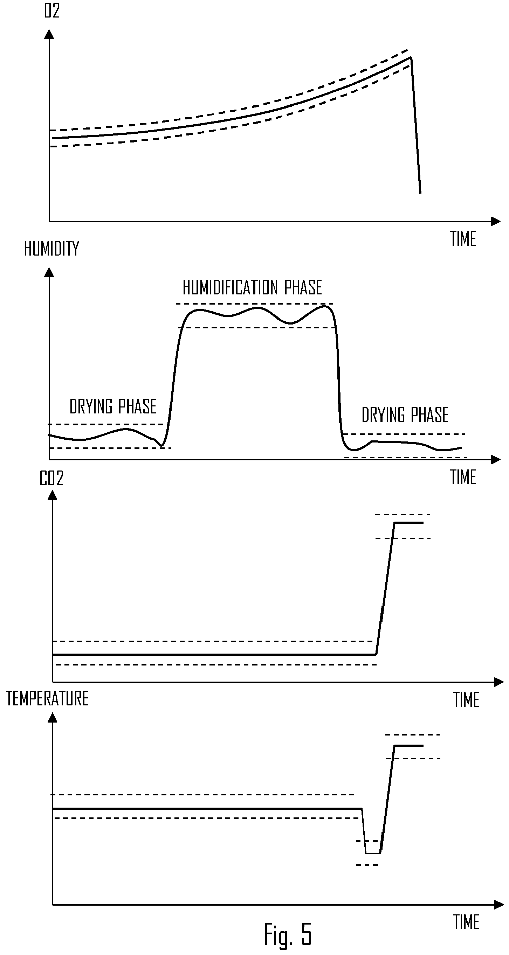

The processes or methods described in Figures 2, 3 and 5 or any of the embodiments thereof may also be carried out in the form of one or more computer processes defined by one or more computer programs. In particular, the analysis of the measurement data and decision on the following control actions may be defined by the computer program. Similarly, the functions of the processing system 140 may be defined by a computer program product stored, read, and executed in the processing system. The computer program(s) may be in source code form, object code form, or in some intermediate form, and it may be stored in some sort of carrier, which may be any entity or device capable of carrying the program. Such carriers include transitory and/or non-transitory computer media, e.g. a record medium, computer memory, read-only memory, electrical carrier signal, telecommunications signal, and software distribution package. Depending on the processing power needed, the computer program may be executed in a single electronic digital processing unit (processor) or it may be distributed amongst a number of processing units. References to computer-readable program code, computer program, computer instructions, computer code etc. should be understood to express software for a programmable processor such as programmable content stored in a hardware device as instructions for a processor, or as configured or configurable settings for a fixed function device, gate array, or a programmable logic device.

It will be obvious to a person skilled in the art that, as the technology advances, the inventive concept can be implemented in various ways. The invention and its embodiments are not limited to the examples described above but may vary within the scope of the claims.

## Claims

1. A method for rearing black soldier fly larvae, comprising:
providing a container (150) with trays (110) for rearing the larvae, the trays comprising the same composition of pre-processed and homogenous biomass of larvae food and black soldier fly larvae;
using one or more of the trays as a reference tray provided with a sensor system (130, 132) to measure at least moisture of the biomass regularly during the rearing;
measuring (200), by using at least one temperature sensor (130, 126), temperature in the reference tray and/or air temperature in the container;
controlling (204, 206), dynamically in response to the measured moisture of the biomass and the measured temperature, temperature and humidity inside the container to maintain the temperature in the biomass within a determined range and to maintain the measured moisture in the reference tray below a level where liquid separate from the biomass;
upon completing the rearing, controlling (310) at least one of the temperature and humidity in the container to dry a remainder of the biomass, frass, and the larvae.

2. The method of claim 1, wherein the biomass is crushed potatoes, and wherein a mass of the black soldier fly larvae, at an age of five to seven days, is about 0.00003 to 0.00004 weight percent of a mass of the pre-processed and homogenous biomass of larvae food.

3. The method of claim 1 or 2, wherein a mass of the reference tray is also monitored and, upon detecting during the rearing that the mass reaches a threshold, determining that the rearing is completed, and triggering said increase of the temperature to dry the trays and harvest the larvae.

4. The method of any preceding claim, further comprising upon completing the rearing: increasing an amount of carbon dioxide in the container to stun the larvae and, thereafter, increasing the temperature to terminate the larvae.

5. The method of claim 4, further comprising providing one or more vessels (400) below the trays to collect larvae that escape from the trays and harvesting the larvae from the vessels after said termination, thus separating the larvae from the biomass.

6. The method of any preceding claim, where an oxygen sensor is provided in the container, the method further comprising controlling the portion of oxygen in the air inside the container such that the portion of oxygen is increased towards the end of the rearing.

7. The method of any preceding claim, wherein temperature inside the container is controlled such that, upon completing the rearing, the temperature is first decreased to stun the larvae and, thereafter, increased to terminate the larvae.

8. The method of any preceding claim, wherein an imaging sensor is directed to monitor a surface of the biomass, and wherein motion of the larvae is monitored by analysing images captured by the imaging sensor, the method further comprising triggering a control action to reduce the moisture upon detecting in the analysis that the motion of the larvae increases a threshold.

9. The method of any preceding claim, wherein humidity control during the rearing follows the following pattern:
during a first time interval from a start of the rearing, the humidity control is drying to prevent the liquid from separating from the biomass;
during a second time interval following the first time interval, the humidity control is humidifying to prevent the biomass from drying;
during a third time interval following the second time interval and associated with completing the rearing, the humidity control is to dry the biomass and the larvae.

10. The method of any preceding claim, wherein oxygen volume control during the rearing follows the following pattern: the relative volume of oxygen in the container is increased towards the end of the rearing and decreased upon completing the rearing.

11. The method of any preceding claim, wherein carbon dioxide volume control during the rearing follows the following pattern: the relative volume of carbon dioxide is increased upon completing the rearing to stun the larvae before carrying out the increase of the temperature.

12. The method of any preceding claim, wherein temperature control during the rearing follows the following pattern: the temperature is maintained within a determined range during the rearing and, upon completing the rearing, the temperature is first decreased below the determined range before said increasing the temperature above the determined range.

13. The method of claim 12 as dependent on claim 11, wherein the temperature is decreased below the determined range before increasing the relative volume of carbon dioxide.

14. The method of any preceding claim, wherein the container comprises, in addition to the reference tray, a plurality of trays for rearing the larvae, wherein the plurality of trays either have dimensions and contents that are identical to dimensions and contents of the reference tray or have dimensions and contents proportional to the dimensions and contents of the reference tray such that they provide the same response to the temperature and humidity control as the reference tray.

15. A system for rearing black soldier fly larvae, comprising:
a container (150);
multiple trays (110) for rearing the larvae;
at least one sensor for one of the trays used as a reference tray, configured to measure at least moisture of a biomass in said reference tray;
at least one temperature sensor (130, 126) to measure temperature in the reference tray and/or air temperature in the container;
means (140) for controlling, dynamically in response to the measured moisture and temperature of the biomass, temperature and humidity inside the container to maintain the temperature in the biomass within a determined range and to maintain the measured moisture in the reference tray below a level where liquid separate from the biomass, and to control, upon completing the rearing, at least one of the temperature and humidity in the container to dry a remainder of the biomass, frass, and the larvae.
